Europäisches Patentamt

European Patent Office

Office européen des brevets

(11) Publication number: **0 288 093**
**A2**

# (12) EUROPEAN PATENT APPLICATION

(21) Application number: **88200122.5**

(22) Date of filing: **26.01.88**

(51) Int. Cl.4: **G01N 33/569 , G01N 33/544 , G01N 33/546 , C07K 15/00 , C12N 15/00**

The title of the invention has been amended (Guidelines for Examination in the EPO, A-III, 7.3).

(30) Priority: **27.01.87 US 6880**

(43) Date of publication of application:
**26.10.88 Bulletin 88/43**

(34) Designated Contracting States:
**AT BE CH DE ES FR GB GR IT LI LU NL SE**

(71) Applicant: **Microgenesys, Inc.**
**400 Frontage Road**
**West Haven New Haven Connecticut 06516(US)**

(72) Inventor: **Cochran, Mark A.**
**415 Brooksvale Avenue**
**Hamden Connecticut 06518(US)**
Inventor: **Smith, Gale E.**
**125 Michael Drive**
**Guilford Connecticut 06437(US)**
Inventor: **Volvovitz, Franklin**
**123 York Street Apt. 18E Crown Court**
**New Haven Connecticut 06516(US)**

(74) Representative: **Smulders, Theodorus A.H.J. et al**
**Vereenigde Octrooibureaux Nieuwe Parklaan 107**
**NL-2587 BP 's-Gravenhage(NL)**

(54) **Strips containing the gp160 envelope protein of the AIDS virus/HIV, and its use in the detection of the AIDS antibody.**

(57) Nitrocellulose strips having recombinant HIV envelope glycoprotein, HIV gp160, spotted and fixed thereon are useful for the detection of antibody to AIDS virus (HIV). A dye, such as coomassie blue, non-covalently attached to the glycoprotein and to the strip is usefully associated with the glycoprotein to identify the location of the glycoprotein spotted and fixed to the strip.

## HIV gp160 AIDS VIRUS ENVELOPE PROTEIN-CONTAINING STRIPS AND USE THEREOF FOR DETECTION OF AIDS ANTIBODY

This application is a continuation-in-part of copending, coassigned application Serial No. 920,197 filed October 16, 1986. The disclosures and teachings of application Serial No. 920,197 are herein incorporated and made part of this disclosure. In .application Serial No. 920,197 there is disclosed and claimed AIDS virus env proteins which are expressed from a recombinant insect virus and use of such proteins in the preparation of vaccines in diagnostic compositions for the detection of AIDS antibody, including techniques employing said proteins for the detection of AIDS virus antibody as well as methods of constructing a recombinant baculovirus expression vector capable of expressing a selected AIDS virus gene and also methods of synthesizing selected AIDS virus polypeptides or proteins and the incorporation of AIDS virus polypeptides or AIDS virus envelope proteins on substrates and use of such substrates for the detection of AIDS antibody.

## BACKGROUND OF THE INVENTION

At present the preliminary testing of human serum or plasma for the presence of antibody to the AIDS virus is done by enzyme linked immunosorbent assay (ELISA). Tested samples which are positive are then subjected to a second test and often this second test is a Western blot analysis. Western blot analysis can be a more sensitive test than ELISA and is often used to help confirm that the tested human product sample has antibodies specific to HIV antigens. However, conventional Western blot analysis is difficult to reproduce.

It is an object of this invention to provide a better, more reliable and convenient procedure for testing human serum and plasma for the presence of antibody to the AIDS virus.

It is another object of this invention to provide test materials or diagnostic materials which are useful for the determination of antibody to AIDS virus.

It is yet another object of this invention to provide analytical test procedures for the detection of AIDS virus antibody in samples to be tested, such as human serum or plasma.

How these and other objects of this invention are achieved will become apparent in the light of the accompanying disclosure. In at least one embodiment of the practices of this invention at least one of the foregoing objects will be achieved.

## SUMMARY OF THE INVENTION

In accordance with this invention there are provided substrates or strips which may be characterized as modified Western blot strips for the detection of antibody to the AIDS virus (HIV). These strips are made up of or consist essentially of a carrier or strip and a predetermined amount of recombinant HIV envelope glycoprotein deposited on or fixed at a desired location or spot onto said substrate or strip. The preferred envelope glycoprotein in the manufacture of the substrates or strips in accordance with this invention is the AIDS virus (HIV) envelope glycoprotein gp160. The strips of this invention, particularly the HIV gp160 strips are useful for in vitro qualitative enzyme-lined immunosorbent assay for the detection of antibody to HIV (AIDS virus) in human serum and are particularly useful for determining or confirming the presence of antibody to HIV in the blood of humans which has been tested positive in a preliminary screening test or in the blood product of humans suspected to have AIDS.

## DETAILED DESCRIPTION OF THE INVENTION

The AIDS virus envelope protein-containing substrates or strips in accordance with this invention, such as the HIV gp strips, are useful in an enzyme-linked immunoassay procedure. In the preparation of the substrates or strips, the HIV envelope glycoprotein or antigen is applied to a spot on the substrate or strip, such as a nitrocellulose strip. The preferred HIV protein gp160 antigen is a glycosylated recombinant protein of approximately 160,000 molecular weight. In the practices of this invention it is preferred that this protein, e.g. HIV gp160, is a recombinant product produced in insect cells from a baculovirus expression vector. Upon production by the insect cells the recombinant HIV gp160 protecin is separated from the cells and purified, usually employing several operations, the last of which is usually an electroelution from preparative SDS-polyacrylamide gels. The gel purified protein is then applied or fixed as a spot or spotted or applied onto substrates os strips, such as nitrocellulose strips, in accordance with this invention.

In the use of these substrates or strips in accordance with this invention for the detection of AIDS virus antibody, human serum samples to be tested are prediluted and added to or brought into contact with the strips. Human antibodies to HIV envelope protein, if present, will bind to the antigen spotted on the strips and are detected by their reaction with a suitable anti-antibody, such as an anti-antibody conjugated to an enzyme, e.g. goat anti-human immunoglobulin conjugated to alkaline phosphatase. In another approach the human AIDS anti-envelope antibodies bound on the strip could be detected with S. aureus protein A conjugated to [125]iodine and autoradiography. These methods are representative of detection techniques for eliciting the presence of antibody bound to the AIDS envelope glycoprotein fixed to the substrate or strips employed in the test. Other procedures involving other enzymes, such as acid phosphatase, B-galactosidase or peroxidase, may be employed, as well as other anti-antibodies and other radioactive labelled reagents attached to anti-antibody or other agents, such as S. aureus protein A, for fixing to the AIDS antibody bound to the glycoprotein fixed to the strip, may be employed.

Western blot analyses of human serum from AIDS patients carried out with the materials and the techniques of this invention and Western blot analyses conventionally carried out at a leading government clinical laboratory have confirmed that the substrates or strips of this invention containing the recombinant HIV antigen gp160 bound thereon and the recombinant HIV antigen gp160, when employed in a conventional Western blot assay, is sensitive and specific for HIV envelope antibodies. These tests have shown that 100% of the sera from individuals diagnosed as having AIDS have antibodies that recognize the recombinant HIV antigen glycoprotein employed in the practices of this invention and there have been no false positives in the sera of humans which have tested negative for AIDS.

As mentioned hereinabove, conventional Western blot is difficult to reproduce. However, this lack of reproducibility and other limitations have been overcome in the practices of this invention. In the preparation of the recombinant HIV envelope glycoprotein, e.g. gp160, the electrophoretic elution of the protein from SDS-PAGE gels and its attachment to the strips, such as nitrocellulose strips or filters, have been divided or separated into two steps. Specifically, the recombinant HIV gp 160 is electroluted from SDS-PAGE gels. Thereupon, a known or measured amount of the eluted purified gp160 antigen is applied to the carrier substrates or strips, such as nitrocellulose strips. An important advantage of preparing the strips in this manner is that a measured or known or constant amount of antigen, gp160 is applied to each carrier strip (± 5% pipetting error). Therefore, the amount of antigen glycoprotein gp160 applied to the strip can be optimized for the purpose of the test. It has been found that 375 ng purified recombinant HIV gp160 added as a spot to the strip yields satisfactory results. Another advantage is that the location of the antigen gp160 on the strip can be controlled. Various amounts of the glycoprotein antigen may be applied for fixing to the strip, such as an amount in the range from about 150 ng to about 800 ng, e.g. 300-500 ng or 200-600 ng, more or less.

Advantageously, other materials or adjuvants or dyes may be employed in association with the purified antigen glycoprotein spotted on the substrate or strip. For example, adjuvants might be employed to improve the bonding or fixing or stability of the glycoprotein antigen on the substrate or strip to which it is applied.

In one special embodiment of the practices of this invention, a dye which is not covalently attached to the antigen glycoprotein is deposited along with or onto the applied glycoprotein. The dye applied along with the antigen glycoprotein is useful in determining where on the strip or substrate the glycoprotein antigen is applied or located. Since the dye is not covalently attached to the protein, it will diffuse off the strip or substrate during incubation when the substrate or strip is employed in an AIDS antibody testing procedure.

The HIV glycoprotein-containing strips of this invention, e.g. the HIV gp160 strips, have been found to be equivalent in specificity to conventional Western blot strips and have exhibited virtually no specific binding with several human sera known to be negative for AIDS antibodies, even when incubated at serum dilutions as low as 1/10. Indeed, it has been found that the strips, particularly HIV gp160 strips in accordance with this invention, are more sensitive than Western blot strips made from the same HIV gp160 protein. This improved sensitivity would appear to be due to the fact that in the practices of this invention, there is produced a relatively high concentration of antigen glycoprotein, gp160, as compared with conventional Western blotting procedures. Further, the applied recombinant HIV gp160 in accordance with this invention will not have been exposed to the denaturing conditions during electroelution (20% methanol and heat) in the preparation of conventional Western blot strips. This factor contributes to the sensitivity of the antigen test strips prepared in accordance with this invention.

In a production operation for carrying out this invention, each lot of the purified recombinant protein, e.g. gp160, is tested for chemical and immunological purity. A sample of the purified recombinant protein gp160 envelope protein, about 5 ug, is electrophoresced on a 10% SDS polyacrylamide gel and either stained with a suitable dye, e.g. coomassie glue, or transferred to a suitable substrate, such as a nitrocellulose strip. The resulting nitrocellulose strip containing the glycoprotein, e.g. gp160, thereon, together with the associated dye, coomassie blue, is then incubated overnight with a 1/1000 dilution of a high positive human AIDS positive reference serum or a 1/1000 dilution of a human AIDS negative control serum. The specifically bound antibody is detected with $^{125}$iodine protein A. A production lot of antigen would be accepted if a single 160,000 molecular weight protein is visible on the stained gel and a single antigen is detected on the Western blot with a positive serum, while showing no reactivity with the negative control serum. The antigen glycoprotein thus tested would be used to prepare the substrates or strips in accordance with this invention. These HIV gp160 strips are then tested against $10^1$ to $10^8$ dilutions of AIDS positive sera and the reference negative control serum and the resulting produced strips would be considered acceptable in the practices of this invention if there is a distinctly positive reaction at a serum dilution of $10^7$ of the positive serum and no non-specific reactivity against the HIV glycoprotein with $10^1$ dilution of the negative control serum. These strips so prepared would be stable for at least 2 months when stored at a low temperature, about -20°C., in a tightly capped container.

The HIV gp spotted substrates or strips in accordance with this invention have been found to be very sensitive for the detection of antibodies to HIV envelope proteins, e.g. gp120 and gp441. For example, AIDS-positive test serum has an end point dilution (the dilution that produces distinctly positive reaction) of from $10^7$ to $10^8$. This means that if sera are being tested with the strips of this invention in 1 ml at a dilution of 1° antibody of $10^2$, contamination with 0.001 ul of a positive sera could result in a false positive reaction. Accordingly, because of this sensitivity of the glycoprotein-containing substrates or strips of this invention, it is important there be no cross-contamination of the test solutions from one strip to another, especially during incubation with the primary human antibody. It would be desirable, therefore, that each strip or substrate during use be incubated in a disposable test tube. Disposable trays (e.g. Accutran disposable 8-well trays from Schleicher & Schuell) are also usefully employed in tests employing the strips of this invention. The strips, e.g. HIV gp160 strips would be incubated in every other well. Reuse of incubation trays or tubes is not recommended unless the tubes or trays are thoroughly washed between each use. For example, the Accutran trays can be immersed in chromic acid and then washed and, when so treated, the trays can be reused to yield satisfactory results.

The strips prepared in accordance with this invention are optimized for overnight incubation with the primary (human serum) antibody, although shorter incubation times can give satisfactory results. The amount of gp160 antigen applied to the strips or substrates may not be optimal for shorter incubations and the indicator dye associated with the glycoprotein antigen may not completely diffuse off the substrate or strip during a shorter assay period.

In the practices of this invention, several considerations should be taken into account before beginning a blotting assay for a given series of 1° and enzyme conjugated 2° antibodies. In over 100 AIDS clinical sera all have given a good positive response at $10^6$ or greater dilution. Dilutions down to 1/10 have also been used with good results, i.e. there is no non-specific binding of antibody at this low dilution. It is currently preferred to use s 1/100 dilution of human serum and in the testing procedure it is recommended that the test be started at that dilution. The optimal dilution of 2° goat anti-human (gamma-chain specific) IgG alkaline phosphatase conjugate varies from manufacturer to manufactuer and lot to lot. 2° antibody from Sigma has been tested and it has been found that dilutions of 12000 to 1/7500 gave satisfactory results. If the 2° antibody (Ab) is too concentrated, there will be a slight discoloration of the entire substrate or strip employed in the test. In the test employed in a dilution series of 2° Ab concentrations, it is preferred to choose the lowest dilution which does not significantly discolor the strips, e.g. nitrocellulose strips. In general, a wide range of both 1° and 2° antibody concentrations could be used with the HIV gp160 strips of this invention with nearly equivalent results.

In the practices of this invention, as indicated hereinabove, various procedures may be employed for carrying out the tests and for detecting the AIDS antibody fixed to the gp antigen bound to the substrate or test strip. In a test procedure employing goat antihuman IgG alkaline phosphatase conjugate for the detec tion of the bound AIDS antibody, the following test procedure is preferred:

## TEST PROCEDURE EMPLOYING ALKALINE PHOSPHATASE

Blocking Step:

Add 1 ml blocking solution (TN + 5% instant milk) to every other well in Accutran or similar trays. Place one HIV gp160 strip in each well and incubate on a rocker platform for 3 minutes. Aspirate the blocking solution and rinse the strips once with TN-T buffer. All incubations are performed at room temperature.

### 1° Antibody Incubation:

Dilute 1/100 the human sera samples into 1 ml TN-T buffer + 1% BSA by adding 10 ul serum to 990 ul buffer. Add 750 ul to each strip and incubate on a rocker platform with gentle rocking overnight (12 hours or longer).

### Wash:

Aspirate off the 1° antibody using a clean pipet for each strip. Wash the strips twice with about 1 ml TN-TN buffer then twice with TN-T buffer, 5 minutes each wash.

### 2° Antibody Conjugate Incubation:

Dilute goat anti-human IgG AP conjugate 1/3000 in TN-T + 1% bovine serum albumin. Add 1 ml to each strip and incubate 1 hours with gentle rocking.

### Wash:

Wash the strips three times with about 1 ml TN-TN buffer, 5 minutes each wash.

### 2° Antibody Conjugate Incubation:

Dilute goat anti-human IgG AP conjugate 1/3000 in TN-T + 1% bovine serum albumin. Add 1 ml to each strip and incubate 2 hours with gentle rocking.

### Wash:

Wash the strips three times with about 1 ml TN-TN buffer, 5 minutes each wash then rinse briefly with about 1 ml alkaline phosphatase buffer.

### Chromagen Reagent:

Make 1 ml chromagen development solution for each strip. For every 10 ml solution add 100 ul BCIP and mix again. Prepare the chromagen reagent just before use. Add 1 ml to each strip and incubate in the dark for 30 minutes. Longer incubations are not recommended. Stop the color development by washing the strips with three or more rinses of deionized.

### Reading the Strips:

The strips should be dried by blotting them on clear filter paper before attempting to read the results. It is recommended that they be lined up, taped on a sheet of paper, labeled, and stored protected from the light.

In another test procedure employing [125] iodine protein for the detection of the bound AIDS antibody, the following procedure is employed:

### TEST PROCEDURE EMPLOYING [125] IODINE PROTEIN A

### Blocking Step:

Add 1 ml blocking solution (TN + 5% instant milk) to every other well in Accutran of similar trays. Place one HIV gp160 strip in each well and incubate on a rocker platform for 30 minutes. Aspirate the blocking solution and rinse the strips once with TN-T buffer. Note: all incubations are performed at room temperature.

### 1° Antibody Incubation:

Dilute 1/100 the human sera samples into 1 ml TN-T buffer + 1% BSA by adding 10 ul serum to 990 ul buffer. Add 750 ul to each strip and incubate on a rocker platform with gentle rocking overnight (12 hours or longer).

## Wash:

Aspirate off the 1° antibody using a clean pipet for each strip. Wash the strips twice with about 1 ml TN-TN buffer then twice with TN-T buffer, 5 minutes each wash.

## Protein A:

Dilute ¹²⁵iodine Protein A (New England Nuclear; NEX-146L immunology grade or equivalent) to 0.1 uCi/ml in TN-T + 1% BSA and add 1 ml to each strip. Incubate with gentle rocking for 2 hours.

## Wash:

Wash the strips three times with about 1 ml TN-TN buffer + 10 mM EDTA, 5 minutes each, dry, and tape to a sheet of filter paper.

## Autoradiography:

Place in X-ray holder with an intensifying screen and expose overnight at -70°C. Develop film as recommended by manufacturer.

In the above-referred test procedures it is recommended that buffers prepared in accordance with this invention be employed as other conditions and buffers may not be as sensitive or reliable. Accordingly, in the test described hereinabove with respect to alkaline phosphatase and ¹²⁵iodine Protein A, buffers having the following compositions are desirably employed.

## BUFFERS:

10x TN
12.1 g Tris base;      0.1 M Tris, pH 8.0
87.7 g NaCl;      1.5 M NaCl
pH 8.0 adjust with HCl
Bring to 1.0 liter with distilled water

TN-T
100.0 ml 10x TN      0.01 M tris, pH 8.0
30.0 ml 10% Tween-20      0.3% Tween-20
Bring to 1.0 liter with distilled water

TN-TN
100.0 ml 10x TN      0.01 M Tris, pH 8.0
30.0 ml 10% Tween-20      0.3% Tween-20
50.0 ml 10% TritonX-100      0.5% TrintonX-100
Bring to 1.0 liter with distilled water

TN + 5% milk
100.0 ml 10x TN      0.01 M Tris, pH 8.0
0.1 g NaAzide      0.01% NaAzide
50.0g Instant Milk Carnation      5.0% Instant Milk
Bring to 1.0 liter with distilled water

TN-T + 1% BSA
100,0 ml 10x TN      0.01 M Tris, pH 8.0
30.0 ml 10% Tween-20      0.3% Tween-20
10.0g Bovine Serum Albumin      1.0% BSA
Bring to 1.0 liter with distilled water

Alk.Phos.
8.4 g NaHCO₃      0.1 M NaCO₃
0.2 g MgCl₂      1.0 mM MgCl₂
pH 9.8, adjust with HCl
Bring to 1.0 liter with distilled water

100 X NBT
300.0 mg nitro blue tetrazolium
(NBT;BioRad170-6532)
10.0 ml 70% dimethylformamide

100 X BCIP
150.0 mg 5-bromo-4-chloro-3-indolyl phosphate
(BCIP; BioRad 170-6539)
10.0 ml 100% dimethylformamide

As will be apparent to those skilled in the art in the light of the foregoing disclosure, many modifications, alterations and substitutions are possible in the practice of this invention without departing from the spirit or scope thereof.

Although emphasis in this disclosure and the practices of this invention has been placed upon the use of a strip as the substrate upon which the HIV glycoprotein is deposited or fixed for the detection of antibody to AIDS virus (HIV), materials other than nitrocellulose and plastic strips may be employed in the practices of this invention. For example, inorganic glass and ceramic surfaces may be employed for deposition and fixing of the deposited glycoprotein thereon. Further, instead of a strip, slide or other surface for deposition of the glycoprotein thereon, there may be employed particulate materials or beads, such as beads of the same material as the aforementioned strips and slides. Particularly useful as beads for the deposition of the HIV glycoprotein thereon are plastic beads, such as polystyrene beads, essentially spherical bead-like particles of a controlled small diameter, such as a diameter in the range 2-500 microns, more or less. These beads may be of uniform diameter or may have varying diameters in the range 2-500 microns, more or less.

The various known techniques for bringing about and/or detecting antigen-antibody bonding or reaction may be employed, such as enzyme-linked immunosorbent assay (ELISA) and solid phase immunoassay. When beads are employed in the practices of this invention for the detection of antibody to AIDS virus (HIV), there would be deposited and fixed on the beads the HIV (AIDS) glycoprotein in a suitable amount. The beads thus contacted or coated with the AIDS virus (HIV) antigen glycoprotein would then be brought into contact with a sample of human serum or plasma for detection of the antibody to AIDS virus (HIV). Thereupon, as indicated herein, the beads having antibody bound to the antigen glycoprotein thereon would be brought into contact with an anti-antibody for detection of the antibody to HIV (AIDS) virus bound to the glycoprotein AIDS antigen on the surface of the beads. A suitable such anti-antibody for the detection of the antibody bound to the beads would be goat anti-human immunoglobulin, such as goat anti-human immunoglobulin conjugated to alkaline phosphatase, acid phosphatase or a peroxidase. The anti-antibody enzyme conjugate bound to the resulting AIDS antibody-antigen complex on the beads would be determined or elicited through the use of a suitable enzyme reactive chromagen reagent. Further, instead of employing an anti-antibody enzyme conjugate, such as anti-antibody alkaline phosphatase conjugate, one could employ an anti-antibody biotin labeled conjugate which would then be contacted by means of an avidin-enzyme complex, such as avidin-alkaline phosphatase or peroxidase conjugate.

Also, particularly important in the practices of this invention is the HIV envelope glycoprotein as antigen. The preferred HIV glycoprotein is gp160, a glycosylated protein of 160,000 molecular weight. Particularly useful is the HIV glycoprotein or antigen produced as a recombinant product from insect cells by way of baculovirus expression vector. The HIV glycprotein thus produced is separated or recovered from insect cells and purified involving electrophoresis and electroelution from preparative SDS-polyacrylamide gel for the production of substantially pure undenatured or native glycoprotein. The resulting gel purified HIV envelope glycoprotein, particularly HIV gp160, is deposited or applied or fixed to the substrates, strips or beads in accordance with this invention. Accordingly, the pure, substantially native or undenatured HIV envelope glycoprotein, in particularly HIV gp160, of this invention is of great importance because of its activity (not having been denatured) and powerful immunogenic effect in addition to its very strong capability of attaching or binding to HIV (AIDS virus) antibody.

## Claims

1. A substrate, carrier or strip useful for the detection of antibody to AIDS virus (HIV) consisting essentially of a carrier or strip and a predetermined amount of recombinant HIV glycoprotein deposited on or fixed to said substrate or strip.

2. A substrate, carrier or strip in accordance with claim 1 wherein said glycoprotein is HIV envelope glycoprotein gp160.

3. A substrate, carrier or strip in accordance with claim 1 or claim 2 wherein said glycoprotein is deposited on or fixed on said substrate, carrier or strip in an amount in the range from about 150 ng to about 800 ng, preferably 200-600 ng, more preferably 300-500 ng.

4. A substrate, carrier or strip in accordance with any of the claims 1-3 wherein said glycoprotein is deposited on or fixed as a spot on said substrate, carrier or strip.

5. A substrate, carrier or strip in accordance with any of the claims 1-4 wherein said glycoprotein is HIV envelope protein gp160 and wherein said glycoprotein is deposited on or fixed on said substrate, carrier or strip in an amount of about 375 ng.

6. A substrate, carrier or strip in accordance with any of the claims 1-5 wherein a dye is admixed or associated with said glycoprotein deposited on or fixed on said substrate, carrier or strip, said dye being non-covalently attached to said glycoprotein and capable of being diffused from said substrate, carrier or strip when said substrate, carrier or strip is incubated in the presence of a sample undergoing test for detection of antibody to AIDS virus (HIV).

7. A substrate, carrier or strip in accordance with claim 6 wherein said dye is coomassie blue.

8. A substrate, carrier or strip in accordance with any of the claims 1-7 wherein said substrate, carrier or strip is a nitrocellulose-containing strip, such as a nitrocellulose strip.

9. A substrate, carrier or strip in accordance with any of the claims 1-8 wherein said substrate, carrier or strip containing said envelope glycoprotein is a synthetic or plastic strip suitable for fixing said deposited envelope glycoprotein to the surface thereof.

10. A substrate, carrier or strip in accordance with any of the claims 1-9 wherein said substrate, carrier or strip is blocked before a sample for the detection of antibody to AIDS virus (HIV) is applied thereto.

11. A substrate, carrier or strip in accordance with any of the claims 1-10 wherein said recombinant HIV envelope protein deposited on or fixed on said substrate, carrier or strip is produced in insect cells by a baculovirus expression vector.

12. A substrate, carrier or strip in accordance with any of the claims 1-11 wherein said antibody to AIDS virus (HIV) is attached to the recombinant HIV envelope glycoprotein deposited on or fixed on said substrate, carrier or strip.

13. A substrate, carrier or strip in accordance with claims 12 wherein additionally said antibody has attached thereto anti-antibody conjugated to an enzyme.

14. A substrate, carrier or strip in accordance with claim 13 wherein said anti-antibody is goat anti-human immunoglobulin.

15. A substate, carrier or strip in accordance with claim 13 or claim 14 wherein said enzyme is alkaline phosphatase.

16. A substrate, carrier or strip in accordance with claim 13 or claim 14 wherein said enzyme is acid phosphatase.

17. A substrate, carrier or strip in accordance with claim 13 or claim 14 wherein said enzyme is a peroxidase.

18. A substrate, carrier or strip in accordance with claim 12 wherein additionally said antibody has attached thereto S. aureus protein A conjugated to $^{125}$iodine.

19. A method for the detection of antibody to AIDS virus (HIV) in a sample of human serum or plasma which comprises depositing or fixing a predetermined or measured amount of recombinant HIV envelope glycoprotein on a substrate, carrier or strip and incubating said substrate, carrier or strip in the presence of a sample of human serum or plasma for the detection of antibody to AIDS virus (HIV) therein so as to fix any of said antibody to said glycoprotein on said substrate, carrier or strip.

20. A method in accordance with claim 19 wherein anti-antibody to said antibody is applied to said substrate, carrier or strip so as to fix said anti-antibody to any of said antibody fixed on said substrate, carrier or strip, said anti-antibody being conjugated to an enzyme for detection.

21. A method in accordance with claim 20 wherein said anti-antibody is goat anti-human immunoglobulin, and said enzyme is a peroxidase, alkaline phosphatase, or acid phosphatase.

22. A method in accordance with claim 20 or claim 21 wherein said substrate, carrier or strip is a nitrocellulose-containing device, such as a nitrocellulose strip, or a plastic device.

23. A method in accordance with any of the claims 20-22 wherein a chromagen is added for the detection of the enzyme conjugated to the anti-antibody fixed to said antibody on said substrate, carrier or strip.

24. A substrate or carrier useful for the detection of antibody AIDS virus (HIV) in accordance with claim 1 wherein said substrate or carrier consists of substantially spherical particles.

25. A substrate or carrier in accordance with claim 24 wherein said particles are plastic particles, preferably polystyrene particles, preferably having a size or diameter in the range from about 2 microns to about 500 microns.

26. Substantially pure undenatured AIDS virus (HIV) glycoprotein.

27. Glycoprotein in accordance with claim 26 wherein said glycoprotein is substantially pure undenatured gp160.

28. Glycoprotein in accordance with claim 26 wherein said glycoprotein is a recombinant glycoprotein produced in insect cells from a baculovirus expression vector.

29. Glycoprotein in accordance with claim 28 wherein said glycoprotein is separated from the insect cells and purified employing electroelution from SDS-polyacrylamide gel.